# EUROPÄISCHE PATENTSCHRIFT

(11) **EP 3 711 718 B1**
(45) Veröffentlichungstag und Bekanntmachung des Hinweises auf die Patenterteilung: **20.10.2021**
(21) Anmeldenummer: 19163907.9
(22) Anmeldetag: 19.03.2019
(51) Int. Cl.: A61F 5/01, A61F 5/058, A61H 1/02

(54) **VERFAHREN ZUR HERSTELLUNG EINER HANDORTHESE**
METHOD FOR MANUFACTURING A HAND ORTHOTIC
PROCÉDÉ DE FABRICATION D'UNE ORTHÈSE DE LA MAIN

(43) Veröffentlichungstag der Anmeldung: 23.09.2020
(73) Patentinhaber: HKK Bionics GmbH, 89081 Ulm (DE)
(72) Erfinder: Hepp, Dominik, 89231 Neu-Ulm (DE); Knobloch, Tobias, 89077 Ulm (DE)
(74) Vertreter: Hentrich Patentanwälte PartG mbB

(56) Entgegenhaltungen:
- EP-A1- 3 263 086
- CN-A- 105 997 319
- DE-A1-102013 001 732
- US-A- 5 178 137
- US-A1- 2017 266 075

## Beschreibung

Die Erfindung betrifft ein Verfahren zur Herstellung einer Handorthese gemäß Anspruch 1. Weiterhin wird auch ein Beispiel für eine Handorthese zum Beugen und/oder Strecken mindestens eines Fingers einer Patientenhand mit einem Fingerabschnitt zur Aufnahme mindestens eines Fingers einer Patientenhand, vorzugsweise der gesamten Patientenhand, und mit einer Schiene zum zumindest teilweise Umgreifen eines Unterarms eines Patienten, sowie mit einem an der Schiene befestigten Krafteinleitungsmittel, das mit mindestens einem dem Finger der Patientenhand zugeordneten Fingersegment gekoppelt ist, wobei das mindestens eine Fingersegment ein mit der Schiene verbundenes Anfangssegment und ein mit dem Krafteinleitungsmittel verbundenes Endsegment aufweist.

In dem in der EP 3 459 505 A1 beschriebenen Herstellungsverfahren wird anhand eines von einer Patientenhand erstellten Abdrucks ein Handschuhabschnitt durch Aufbringen von Silikon auf den Abdruck erstellt. Die Physiologie des Unterarms des Patienten wird mittels eines Scans der den Handschuhabschnitt tragenden Patientenhand erfasst und ein Modell erstellt. Anhand dessen wird mittels eines generativen Herstellungsverfahrens eine Schiene erzeugt. Nachteilig ist hierbei, dass der Patient bei den meisten Schritten des Herstellungsverfahrens physisch anwesend sein muss, was die Herstellung der Handorthese aufwendiger gestaltet. Die EP 3 459 505 A1 beschreibt zudem eine Handorthese. Diese Orthese hat sich gut bewährt.

Die EP 3 263 086 A1 beschreibt eine Handorthese zum Beugen und/oder Strecken mindestens eines Fingers einer Patientenhand. Weiterhin ist ein Fingerabschnitt zur Aufnahme mindestens eines Fingers einer Patientenhand und eine Schiene, die den Unterarm des Patienten zumindest teilweise umgreift, vorgesehen. Die Fingersegmente sind mit einem Krafteinleitungsmittel und mit der Schiene gekoppelt

Die US 2017/0266075 A1 beschreibt eine als ein Handschuh gebildete Handorthese mit einem Befestigungsgurt.

Die DE 10 2013 001 732 A1 (D3) beschreibt ein Herstellungsverfahren für eine Leit- Stützstruktur, insbesondere für eine Bein- oder Knieorthese. Dabei wird ein Körperteil eines Menschen bezüglich einer als 3D Scanner gebildeten Erfassungseinrichtung ausgerichtet und aus unterschiedlichen Positionen erfasst. Aus dem 3D Scan wird ein 3D Modell der Leit-Stützstruktur mittels eines generativen Fertigungsverfahrens erzeugt. Nachteilig ist hier, dass der Patient für die Erstellung des 3D Modells physisch anwesend sein muss und der 3D Scan aus unterschiedlichen Positionen für den Patienten zeitaufwendig ist.

Die CN 105 997 319 A beschreibt ein Verfahren zur Herstellung einer Schiene oder eines Gipses im Falle eines Bruchs, wobei ein 3D-Scan von der gebrochenen Stelle des Patienten erstellt wird. Anhand des 3D-Scans und anhand eines durch den 3D-Scan gebildeten Modells wird eine Schiene oder ein Gips mittels eines generativen Fertigungsverfahrens erstellt, d.h. der Patient muss ebenfalls für die Erstellung des 3D Modells aus dem 3D Scan physisch anwesend sein.

Es ist daher Aufgabe der vorliegenden Erfindung, ein Verfahren zur Herstellung einer Handorthese bereitzustellen, welches weitestgehend unabhängig vom Patienten ablaufen kann.

Die dem Verfahren zugrunde liegende Aufgabe der Erfindung wird durch das Verfahren mit den Merkmalen des Anspruchs 1 gelöst.

Das Verfahren zur Herstellung einer Handorthese, die einen Fingerabschnitt zur Aufnahme einer Patientenhand, eine Schiene zum zumindest teilweise Umgreifen eines Unterarms eines Patienten und mindestens ein mit einem Krafteinleitungsmittel gekoppeltes Fingersegment aufweist zum Beugen und/oder Strecken mindestens eines Fingers einer Patientenhand umfasst insbesondere die folgenden Schritte:
- Erstellen eines Abdrucks mindestens eines Fingers einer Patientenhand und zumindest eines Teils eines Unterarms des Patienten, vorzugsweise Erstellen eines Abdrucks aller Finger oder der gesamten Patientenhand und vorzugsweise des gesamten Unterarms des Patienten,
- Erstellen eines Fingerabschnitts anhand des Abdrucks oder anhand einer auf dem Abdruck basierenden Nachbildung des mindestens eines Fingers der Patientenhand, vorzugsweise aller Finger der Patientenhand,
- Einbetten von mindestens einem Fingersegment in die der Handoberfläche entsprechende Seite des Fingerabschnitts,
- Erfassen der Physiologie des Unterarms des Patienten mittels mindestens einer Aufnahme, vorzugsweise eines 3D-Scans von dem Abdruck oder von einer auf dem Abdruck basierenden Nachbildung des Unterarms jeweils zusammen mit dem Fingerabschnitt und Erstellen eines digitalen 3D-Modells anhand der mindestens einen Aufnahme, vorzugsweise anhand des 3D-Scans,
- Erzeugen einer Schiene auf Grundlage des erstellten digitalen 3D-Modells,
- Befestigen von mindestens einem Krafteinleitungsmittel auf oder in die Schiene,
- Befestigen eines proximalen Endes des mindestens einen Fingersegments an einem distalen Ende der Schiene und
- Koppeln des mindestens einen Krafteinleitungsmittels mit dem mindestens einen Fingersegment, vorzugsweise im Bereich des distalen Endes.

Das Erfassen der Physiologie des Unterarms des Patienten mittels der mindestens einen Aufnahme, insbesondere mittels des 3D-Scans des Abdrucks oder der auf dem Abdruck basierenden Nachbildung des Unterarms zusammen mit dem Fingerabschnitt ermöglicht es, die Handorthese weitgehend unabhängig vom Patienten herzustellen.

Der Fingerabschnitt kann ein Bestandteil eines Handschuhabschnitts sein. Der Fingerabschnitt kann außerdem aus einem einzelnen Fingerling oder einer Mehrzahl an Fingerlingen zur Aufnahme einzelner Finger einer Patientenhand gebildet sein. Es besteht die vorteilhafte Möglichkeit, dass die Fingerlinge zu einem monolithisch gebildeten Fingerabschnitt oder zu einem aus dem Fingerabschnitt monolithisch gebildeten Handschuhabschnitt zusammengefasst sind. Ferner ist die Möglichkeit gegeben, dass zwei oder mehr der Fingerlinge zu einem gemeinsamen Fingerabschnitt verbunden sind, während mindestens ein weiterer Fingerling getrennt von den verbundenen Fingerlingen vorliegt.

Der Abdruck kann dabei vorzugsweise ein Negativ des Fingers und/oder der Patientenhand und/oder des Unterarms bilden, woraus dann eine als Positiv zu erachtende Nachbildung generiert wird, die für die weiteren Verfahrensschritte zur Herstellung der Handorthese genutzt werden kann.

Alternativ kann der Abdruck als ein Positiv verwendet werden, das unmittelbar für die weiteren Verfahrensschritte zur Verfügung steht. Auf die Erstellung einer Nachbildung oder eines physischen Modells kann dabei verzichtet werden.

Der Abdruck wird dabei bevorzugt mehrteilig insbesondere durch zwei Schalen erzeugt. Dabei können die mindestens zwei Schalen des Abdrucks nach dem Erstarren an der Innenseite miteinander verbunden und somit geschlossen also zusammengefügt werden. Alternativ können von der Patientenhand und vom Unterarm getrennte Abdrücke erstellt werden, die im Anschluss zusammengefügt werden. In diesem Zusammenhang ist es insbesondere vorteilhaft, wenn der Abdruck ein Gipsabdruck ist. Ebenso ist es bevorzugt wenn die auf dem Abdruck basierende Nachbildung des mindestens einen Fingers der Patientenhand und/oder des Unterarms aus Gips erstellt ist.

Die Aufnahme kann ein 3D-Scan aber auch jedes andere 3D-bildgebende Verfahren sein. Alternativ zu einem 3D-Scan kann die Aufnahme auch durch eine Kamera - vorzugsweise eine 3D-Kamera oder auch eine Stereokameraerstellt werden und anhand dieser Aufnahme ein digitales 3D-Modell generiert oder auch errechnet werden. In einer weiteren alternativen Ausführungsform können auch eine oder mehrere Kameras eine Aufnahme des Abdrucks oder der auf dem Abdruck basierenden Nachbildung des Unterarms zusammen mit dem Handschuhabschnitt aus unterschiedlichen Blickwinkeln erstellen. Ein 3D-Modell kann dann beispielsweise mittels Triangulation erstellt oder errechnet werden. Alternativ sind ist die Erstellung eines digitalen 3D-Modells auch mittels strukturierter Beleuchtung, ,mit Hilfe einer Time of Flight-Kamera (TOF) oder mittels Interferometrie möglich.

Insbesondere ist es vorteilhaft, wenn der Abdruck unter einer gebeugten Stellung der Patientenhand erstellt wird. Unter einer gebeugten Stellung wird insbesondere eine greifende Stellung verstanden. Diese wird erreicht mittels Extension des Handgelenks um 10 Grad bis 30 Grad, bevorzugt 15 Grad bis 20 Grad und/oder mittels der Streckung (maximal 10 Prozent Flexion) des MCP-Gelenks (Metacarpophalangealgelenk) und/oder mittels einer Beugung des PIP-Gelenks (proximales Interphalangealgelenk) und/oder des DIP-Gelenks (distales Interphalangealgelenk) um 20 Grad bis 50 Grad und/oder indem der Daumen in einer Oppositionsstellung oder in einer Lateralstellung ist. Insbesondere ist es bevorzugt, wenn alle Finger in leichter Spreizung zueinander angeordnet sind, d.h. zwischen dem Zeige-, Mittel- und Ringfinger sollte ein Abstand oder ein Spalt von mindestens 5 mm auf Höhe des PIP vorhanden sein. Der Ringfinger und der kleine Finger können näher beieinander liegen, also einen Abstand weniger als 5mm zum benachbarten Finger aufweisen.

In diesem Zusammenhang ist es insbesondere vorgesehen, dass die gebeugte Stellung mittels einer Positioniervorrichtung gehalten wird. Dies stellt die optimale Haltung der Hand bzw. der Finger sicher und erleichtert das Erstellen des Abdrucks. Eine geeignete Positioniervorrichtung wäre ein Klotz oder ein Keil oder verschiedene miteinander kombinierbare Keile. In einer alternativen Ausführungsform kann die Haltevorrichtung auch eine kugelförmige oder teilkugelförmige Struktur, beispielsweise ein Ball sein, der von der Patientenhand ergriffen wird.

Ist jedoch die eingesetzte die Positioniervorrichtung als eine teilkugelförmige Struktur gebildet, so kann die gekrümmte Oberfläche dazu genutzt werden, um die gekrümmten Gelenke des Patientenfingers abzubilden, wobei die ungekrümmte planare Oberfläche der teilkugelförmigen Struktur genutzt werden kann, um die gestreckten Gelenke des Patienten abzubilden.

Vorteilhafterweise wird der Handschuhabschnitt mittels eines Auftragens von Silikon auf den Abdruck erstellt. Dabei kann das Auftragen von Silikon durch das Auftragen von mehreren Lagen an Silikon erfolgen. In einer alternativen Ausführungsform können mittels eines generativen Verfahrens an den Abdruck angepasste Formen erstellt werden und die Formen im Anschluss mit Silikon gefüllt werden. Das derart geformte Silikon kann dann im Anschluss auf den Abdruck aufgebracht werden. Alternativ kann das Silikon auch im 3D-Druckverfahren hergestellt und geformt sein, was einen höheren Automatisierungsgrad zur Herstellung der Handorthese bereithält.

Weiterhin ist es von Vorteil, wenn verschiedene Silikone mit unterschiedlichen Härtegraden zum Erstellen des Fingerabschnitts, vorzugsweise des Handschuhabschnitts verwendet werden. Dies ermöglicht es, Teile des Fingerabschnitts, insbesondere eines Handschuhabschnitts die einer Beugung oder Streckung ausgesetzt sind, oder die mit der Hand in Berührung kommen mit einem ersten Silikon und Teile des Fingerabschnitts, insbesondere des Handschuhabschnitts, die hohen Belastungen ausgesetzt sind, mit einem zweiten Silikon zu erstellen, wobei das erste Silikon einen geringeren Härtegrad als das zweite Silikon aufweist. Insbesondere im Bereich der Einbettung des mindestens einen Fingersegments ist es daher bevorzugt Silikon mit einem weicheren Härtegrad (beispielsweise Shore A 15 bis 25) zu verwenden. Zur Versteifung von PIP- und/oder DIP-Gelenken und/oder zur Erstellung von stabilen Zugringen an Eintrittsöffnungen für die Fingersegmente ist es dagegen von Vorteil, einen Silikon mit einem härteren Härtegrad im Bereich von Shore A 50 bis Shore A 70, bevorzugt Shore A 60 zu verwenden. Es besteht zur Erhöhung der Stabilität außerdem die Möglichkeit, dass anstelle eines einen höheren Härtegrad aufweisenden Silikons andere, insbesondere reaktionträge, Versteifungsmittel in das weichere Silikon eingearbeitet oder eingebettet werden. Hierbei kommen Stifte aus Keramik und Metall oder auch Keramiknetze in Betracht.

Zum Stabilisieren und/oder Versteifen des PIP-Gelenks und/oder des DIP-Gelenks kann es zudem von Vorteil sein, zusätzlich in das Silikon ein Material einzubetten, welches gegenüber dem Silikon einen höheren Härtegrad aufweist.

Weiterhin ist es bevorzugt, wenn das mindestens eine Fingersegment in das Silikon mittels einer Silikonstanze eingebettet wird. Dadurch kann eine exakt vordefinierte Menge an Silikon bereitgestellt und verwendet werden.

Darüber hinaus ist es vorgesehen, dass der Silikonfingerabschnitt oder der mehrere Fingerabschnitte umfassende Silikonhandschuh unter Hitzeeinwirkung, beispielsweise in einem Ofen vernetzt wird.

Insbesondere ist es bevorzugt, wenn die Länge des mindestens einen Fingersegments an die Länge eines Fingers des Patienten angepasst wird durch Anordnen und Koppeln von einem oder von mehreren Modulsegmenten zwischen einem mit der Schiene gekoppelten Anfangssegment und einem mit dem Krafteinleitungsmittel verbundenen Endsegment. Durch die Kopplung einer unterschiedlichen Anzahl von Modulsegmenten zwischen dem Anfangssegment und dem Endsegment ist es möglich Fingersegmente unterschiedlicher Länge herzustellen.

Dabei ist es insbesondere vorteilhaft, wenn das Modulsegment als ein Linearsegment oder als ein Gelenksegment gebildet ist und wenn die Länge des mindestens einen Fingersegments des Fingerabschnitts oder des Handschuhabschnitts durch eine Kombination von einem oder von mehreren Gelenksegmenten mit einem oder mit mehreren Linearsegmenten angepasst wird. Die Gelenksegmente ermöglichen dabei eine Kippbewegung während die Linearsegmente eine Translationsbewegung und insbesondere eine Dehnung und ein Ausgleich der Längenänderung der Haut auf einer Fingeraußenseite ermöglichen. Jedes Fingersegment weist also ein Anfangssegment, ein Endsegment und mindestens ein Gelenksegment sowie mindestens ein Linearsegment auf.

Für den Einsatz der Handorthese bei Menschen mit besonders kleinen Fingern, insbesondere mit kleinen Daumen, ist es außerdem möglich, dass zwischen dem Anfangssegment und dem Endsegment lediglich ein Gelenksegment eingebunden oder eine gekoppelte Mehrzahl an Gelenksegmenten eingebunden sind.

Die Physiologie des Unterarms und dadurch das digitale 3D-Modell kann in einer Ausführungsform mittels der mindestens einen Aufnahme, insbesondere mittels des 3D-Scans des Abdrucks oder der auf dem Abdruck basierenden Nachbildung des Unterarms zusammen mit dem Fingerabschnitt oder dem Handschuhabschnitt und mit einer auf zumindest Teilen des Fingerabschnitts oder des Handschuhabschnitts und/oder zumindest Teilen des Abdrucks oder der einen auf dem mindestens einen Abdruck basierenden Nachbildung aufgebrachten Polsterung erfasst werden. Insbesondere ist die Polsterung zumindest auf den mit der Hand in Berührung kommenden Teilen des Fingerabschnitts oder des Handschuhabschnitts aufgebracht. In dieser Ausführungsform wird folglich zunächst das Silikon auf den Fingerabschnitt oder den Handschuhabschnitt und im Anschluss auf das Silikon die Polsterung aufgebracht. Schließlich wird das 3D-Modell anhand der mindestens einen Aufnahme, vorzugsweise anhand des 3D-Scans erstellt. Die Aufnahme kann auch durch eine oder mehrere Kameras erstellt und das 3D-Modell anhand von einer oder mehrerer Aufnahmen erzeugt oder errechnet werden. Dabei kann die Kamera eine 2D-Kamera oder eine 3D-Kamera oder eine Stereokamera sein. Möglich ist es ebenfalls, das digitale 3D-Modell anhand von struktureller Beleuchtung oder Time of Flight-Messungen oder Triangulation zu erstellen oder zu errechnen.

Alternativ kann bei einem ohne Polsterung erstellten 3D-Modell, diesem Modell ein Offset für das Aufbringen einer Polsterung hinzugefügt werden. Der Offset kann dabei der Höhe der später aufgebrachten Polsterung entsprechen oder in einer alternativen Ausführungsform auch geringer als die Höhe der Polsterung gewählt werden. Dadurch wird beim Aufbringen der Polsterung auf den Fingerabschnitt oder den Handschuhabschnitt und auf die Schiene das Spiel zwischen der Handorthese und der Hand geringer, so dass die Handorthese fester und sicherer an der Hand anliegt.

In einer weiteren alternativen Ausführungsform ist es möglich, dass der Offset die spätere Polsterung berechnet bzw. extrahiert und so im Anschluss automatisch ein Zuschnitt der Polsterung durch ein automatisiertes Verfahren hergestellt werden kann; ihre Formgebung basiert auf dem mit Offset versehenen 3D-Modell.

Vorzugsweise kann die aufzubringende Polsterung auch eine direkt generativ, insbesondere mittels 3D-Druck gefertigte Polsterung sein, deren Formgebung auf dem mit Offset versehenen 3D-Modell basiert.

Es besteht zudem die Möglichkeit, dass basierend auf dem 3D-Modell zuerst eine Schablone für eine, insbesondere manuell, zuzuschneidende Polsterung erstellt und/oder ausgegebenen wird, und dass dann die Polsterung anhand der erstellten Schablone zugeschnitten und bereitgestellt wird bevor sie auf den Fingerabschnitt bzw. den Handschuhabschnitt und/oder die Schiene aufgebracht wird. Die Schablone ist vorzugsweise aus Papier oder aus Pappe gebildet und setzt somit nachwachsende Rohstoffe ein.

Die Polsterung wird auf zumindest einen Teil eines Handrückens des Fingerabschnitts oder des Handschuhabschnitts und/oder auf zumindest einen Teil der Schiene aufgebracht. In einer bevorzugten Ausführungsform werden der gesamte Fingerabschnitt oder der gesamte Handschuhabschnitt und die gesamte Schiene gepolstert. Dies erhöht den Tragekomfort der Handorthese.

Es hat sich zudem als sinnvoll erwiesen, wenn vor dem Erfassen der Physiologie des Unterarms mittels der mindestens einen Aufnahme mindestens eine Markierungslinie oder eine Mehrzahl von Markierungspunkten zur Festlegung einer späteren Kontur oder den Randverlauf der Schiene auf den Abdruck oder auf die auf dem Abdruck basierende Nachbildung aufgebracht wird. Es trägt der Passgenauigkeit der späteren Handorthese bei, wenn zusätzlich vor dem Erfassen die Polsterung aufgebracht wurde.

Um den Sitz der Handorthese an der Hand bzw. an dem Unterarm zu verbessern kann dem digitalen 3D-Modell mindestens eine Palmarstütze und/oder mindestens ein Unterarmbügel hinzugefügt werden. Die Palmarstütze und/oder der Unterarmbügel können dann bevorzugt gemeinsam mit der Schiene auf Grundlage des erstellten 3D-Modells erzeugt werden. Eine Mehrzahl von an die Schiene angeformten Palmarstützen und/oder Unterarmbügeln ist für einen besonders sicheren Sitz der Handorthese an den Gliedmaßen des Patienten ebenfalls sinnvoll.

Dabei ist es insbesondere von Vorteil, wenn die Schiene und/oder die Palmarstütze und/oder der Unterarmbügel mittels eines generativen Fertigungsverfahrens erzeugt werden. Dies kann insbesondere durch ein 3D-Druckverfahren erfolgen. Geeignete generative Fertigungsverfahren sind Lasersintern, Laser- oder Siebdruck, Laserstrahlschmelzen Elektronenstrahlschmelzen, Fused layer Modeling, Multi-Jet Modeling, Poly-Jet Modeling, Layer laminated manufacturing, Digital light processing.

Die Krafteinleitungsmittel liegen vorzugsweise als insbesondere einzeln ansteuerbare Servomotoren vor. Das dem betreffenden Fingersegment zugewiesene Krafteinleitungsmittel ist mit einem ersten Ende eines Kopplungselements, insbesondere mit dem ersten Ende eines Drahtes, vorzugsweise eines Nitinoldrahts, verbunden, dessen anderes Ende mit dem Endsegment verbunden ist. Die Krafteinleitungsmittel werden in Aussparungen der Schiene befestigt.

Um eine glatte Oberfläche der Handorthese bereitzustellen, ist es daher in einem weiteren, insbesondere abschließenden Verfahrensschritt von Vorteil, wenn eine Abdeckung, vorzugsweise eine Abdeckhaube oder ein Cover auf die dem Unterarm abgewandte Oberfläche der Schiene aufgebracht und vorzugsweise mit der Schiene verschraubt wird. Diese verdeckt dann die oftmals auch empfindlichen Krafteinleitungsmittel bzw. Servomotoren.

Bei der Handorthese sind zwischen dem Anfangssegment und dem Endsegment mindestens ein Gelenkssegment zur Kippverbindung mit dem benachbarten Segment, vorzugsweise auch mindestens ein Linearsegment mit begrenzter axialer Bewegbarkeit angeordnet. Jedes der Fingersegmente besteht somit aus einem Anfangssegment, einem Endsegment und mindestens einem dazwischenliegenden Gelenksegment, vorzugsweise zusätzlich aus mindestens einem zwischen dem Anfangssegment und dem Endsegment liegenden Linearsegment. Durch die Gelenksegmente wird ein Beugen bzw. eine Kippbewegung oder eine Schwenkbewegung der Fingersegmente ermöglicht, wobei die Schwenkbewegung zumindest einen Winkel von 10 Grad bis 20 Grad - bevorzugt 18 Grad ermöglicht. Die Linearsegmente sorgen für eine Translationsbewegung also auch insbesondere für eine Dehnung und den Ausgleich der Längenänderung der Haut auf der Fingeraußenseite. Dadurch kann die Länge oder den Abstand zwischen einzelnen Gelenkssegmente erhöht werden, um die Länge der Fingersegmente an die Länge der Finger des Patienten anzupassen.

Vorteilhafterweise weisen die Segmente an einer ersten Stirnseite mindestens eine Bajonettkulisse und an einer zweiten Stirnseite mindestens einen zu der Bajonettkulisse korrespondierenden Bajonetthaken auf zur Bildung einer ein Bajonettgelenk bereitstellenden Bajonettverbindung zwischen benachbarten Segmenten.

Insbesondere ist es vorgesehen, dass die Linearsegmente und die Gelenksegmente mehrere, vorzugsweise vier gleichmäßig über den Umfang verteilte Bajonetthaken und mehrere, vorzugsweise vier korrespondierende Bajonettkulissen aufweisen. Dabei weisen die fingerabschnittabgewandten, oberen Bajonetthaken der Gelenksegmente weniger bis kein Spiel in den korrespondierenden Bajonettkulissen auf, während die fingerabschnittabgewandten, oberen und die fingerabschnittzugewandten, unteren Bajonettkulissen der Linearsegmente ein Spiel, bevorzugt dasselbe Spiel, aufweisen und dadurch eine Translation, mithin also eine geradlinige Verschiebung der Bajonetthaken in den Bajonettkulissen bereitstellen.

Um eine Kippbewegung zu ermöglichen, weisen die fingerabschnittabgewandten, oberen Bajonettkulissen des Gelenksegments zudem jeweils einen Vorsprung auf, der mit einer abgewinkelten Vorsprungsfläche ausgebildet ist. Die fingerabschnittabgewandten, oberen Bajonetthaken und die fingerabschnittzugewandten, unteren Bajonetthaken weisen bei den Gelenksegmenten zudem vorzugsweise dasselbe geringe Spiel auf, wodurch eine Kippbewegung der Gelenksegmente ermöglicht, aber eine Translationsbewegung zumindest nahezu vollständig unterbunden ist.

Im Folgenden wird die Erfindung an in der Zeichnung dargestellten Ausführungsbeispielen näher erläutert; es zeigen:
- Fig. 1: eine perspektivische Ansicht einer Handorthese,
- Fig. 2: eine perspektivische Ansicht der Schiene mit einer Palmarstütze und einem Unterarm,
- Fig. 3: eine perspektivische Ansicht eines Fingersegments,
- Fig. 4a: eine perspektivische Ansicht eines Anfangssegments,
- Fig. 4b: eine Schnittansicht der Figur 4c,
- Fig. 4c: eine schienenzugewandte Ansicht des Anfangssegments,
- Fig. 4d: eine schienenabgewandte Ansicht des Anfangssegments,
- Fig. 5a: eine perspektivische Ansicht eines Gelenksegments,
- Fig. 5b: eine Schnittansicht der Figur 5c,
- Fig. 5c: eine Vorderansicht des Gelenksegments,
- Fig. 5d: eine Hinteransicht des Gelenksegments,
- Fig. 6a: eine perspektivische Ansicht des Linearsegments,
- Fig. 6b: eine Schnittansicht der Figur 6c,
- Fig. 6c: eine Vorderansicht des Linearsegments,
- Fig. 6d: eine Hinteransicht des Linearsegments,
- Fig. 7a: eine perspektivische Ansicht des Endsegments,
- Fig. 7b: eine Schnittansicht der Figur 7d,
- Fig. 7c: eine dem benachbarten Segment zugewandte Seite des Endsegments,
- Fig. 7d: ein auf das freie Ende des Endsegments schauende Darstellung und
- Fig. 8: eine schematische Darstellung des Verfahrens zur Herstellung einer Handorthese.

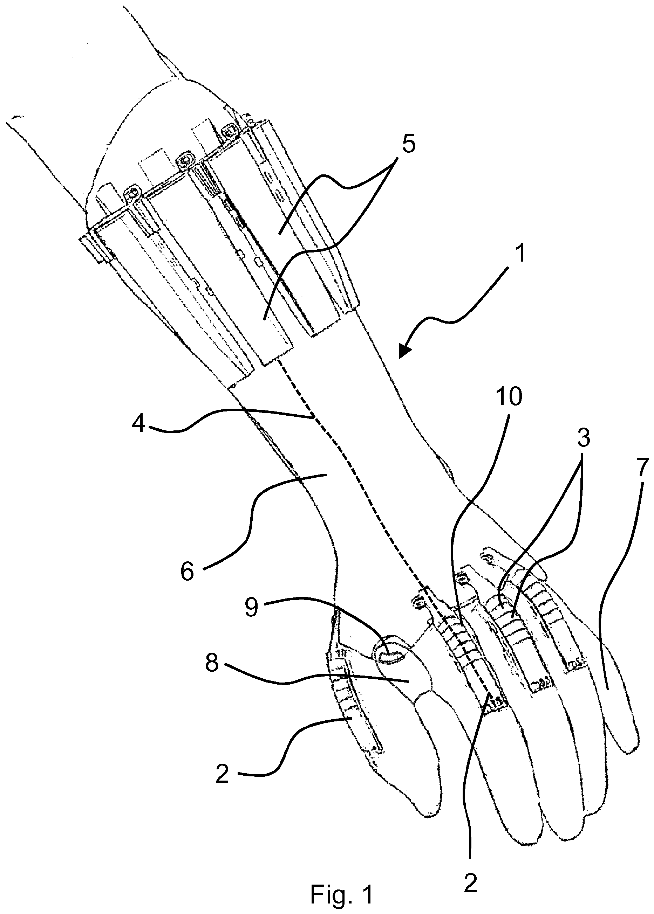

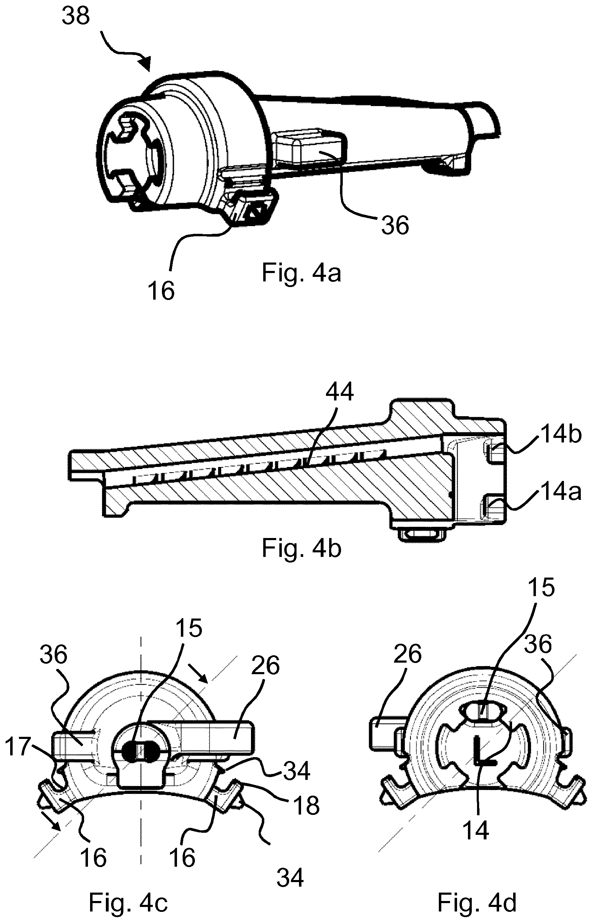

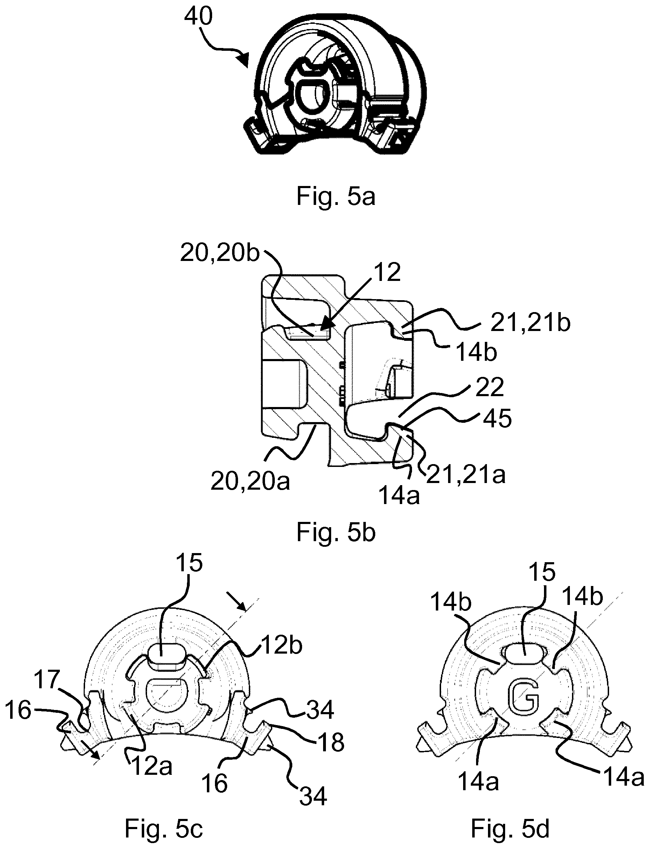

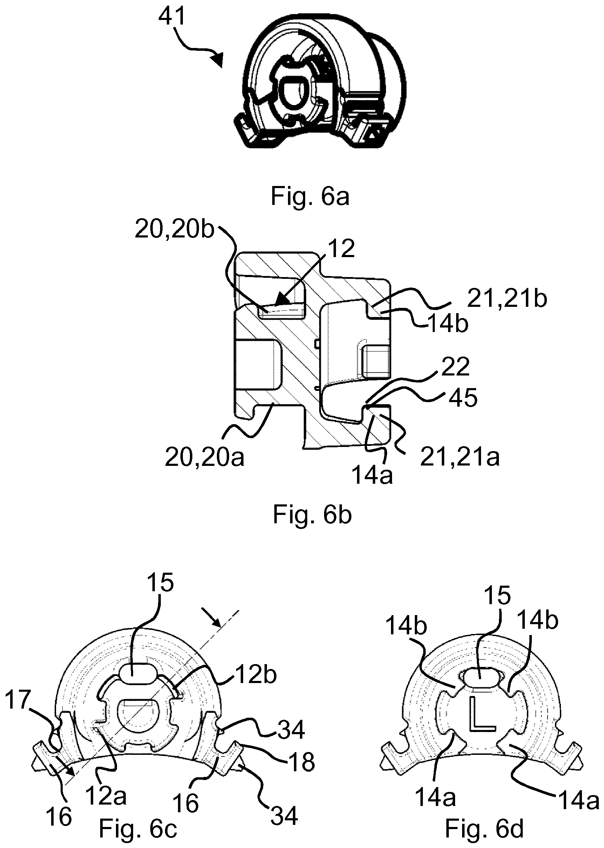

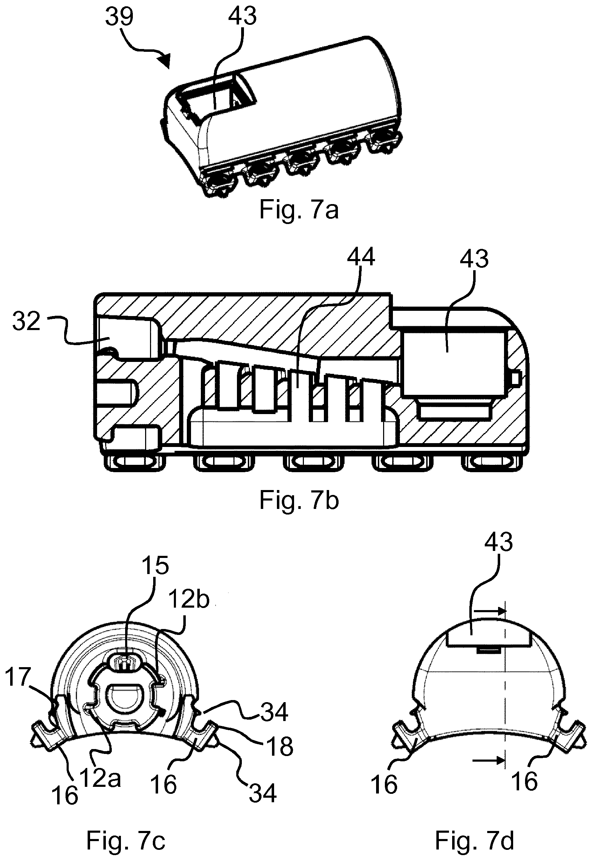

Figur 1 zeigt in einer perspektivischen Ansicht eine Handorthese 1 zum Beugen und/oder Strecken mindestens eines Fingers einer Patientenhand. Die Handorthese 1 umfasst dabei einen Fingerabschnitt, vorliegend einen Handschuhabschnitt 7 zur Aufnahme einer Patientenhand und eine Schiene 6 zum zumindest teilweisen Umgreifen eines Unterarms eines Patienten, sowie eine Mehrzahl von an der Schiene 6 befestigten Krafteinleitungsmitteln 5. Die Krafteinleitungsmittel 5 sind jeweils mit einem Fingersegment 2 gekoppelt, welches einem der Finger der Patientenhand zugeordnet ist. Das Fingersegment 2 weist ein mit der Schiene 6 verbundenes Anfangssegment 38 (Fig. 4a-4d) und ein mit dem Krafteinleitungsmittel 5 verbundenes Endsegment 39 (Fig. 7a-7d) auf. Zwischen dem Anfangssegment 38 und dem Endsegment 39 sind mindestens ein Gelenkssegment 40 (Fig. 5a-5d) zur Kippverbindung mit einem der benachbarten Segmente 38, 39, 40, 41 und mindestens ein Linearsegment 41 (Fig. 6a-6d) mit beidseits begrenzter axialer Bewegbarkeit angeordnet. Die Linearsegmente 41 und die Gelenksegmente 40 sind zusammengefasst als Modulsegmente 3 in Figur 1 gekennzeichnet. Die Fingersegmente 2 sind dabei jeweils an ihren distalen Enden mittels als Drähte geformten Kopplungselemente 4 mit einem als Servomotor gebildeten Krafteinleitungsmittel 5 verbunden, dass an der am Unterarm des Patienten befestigbaren Schiene 6 befestigt ist. Die Kopplungselemente 4, von denen in der Figur 1 lediglich eines gestrichelt illustriert ist, sind dabei sowohl in der Schiene 6 als auch in den Fingersegmenten 2 verdeckt, bevorzugt in einem mehrlumigen, insbesondere doppellumigen Schlauch, geführt und angeordnet.

Figur 1 kann man entnehmen, dass der Fingerabschnitt, vorliegend der Handschuhabschnitt 7 dem distalen Ende der Schiene 6 zugeordnet ist und dass die Fingersegmente 2 an dem Handschuhabschnitt 7 befestigt sind. Wird nun von dem Krafteinleitungsmittel 5 über die Kopplungselemente 4 eine Zug- oder eine Druckkraft auf das Fingersegment 2 ausgeübt, so sorgt dies im Fall einer Zugkraft für ein Strecken im Fall einer Druckkraft für ein Beugen der Fingersegmente 2 und damit letztlich auch der damit verbundenen Finger. Zur Stützung der Handinnenfläche ist mit der Schiene 6 eine Palmarstütze 8, vorzugsweise einstückig, verbunden, welche den Handschuhabschnitt 7 auf dessen der Handfläche zugewandten Seite umgreift und die den Handschuhabschnitt 7 an dem distalen Ende der Schiene 6 mittels einer zusätzlichen lösbaren Rastverbindung 9 befestigt. Alternativ oder ergänzend kann auch das zweite Ende der Palmarstütze 8 fest, mithin einstückig mit der Schiene 6 verbunden sein, so dass die Schiene 6 mit der Palmarstütze 8 eine Öffnung für den Durchtritt des Daumens der Patientenhand bereitstellen.

Die einzelnen Segmente 38, 39, 40, 41 des Fingersegments 2 sind bei der in der Figur 1 dargestellten Handprothese 1 durch eine Bajonettverbindung 10 miteinander verbunden.

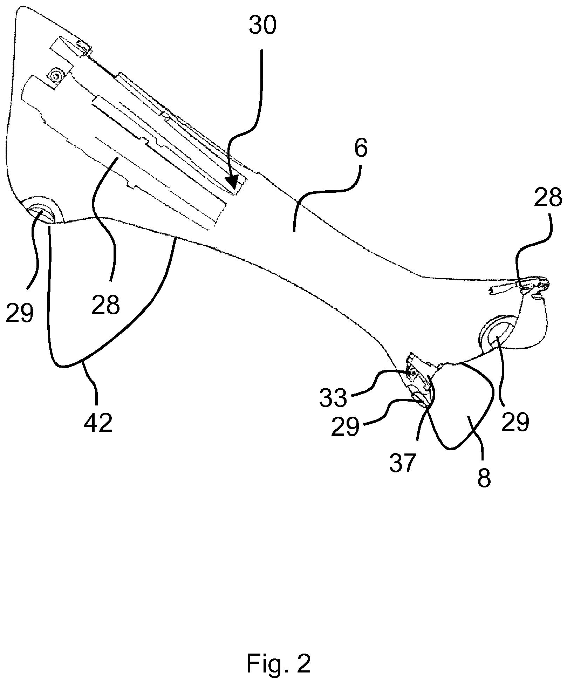

Figur 2 zeigt die Schiene 6 in einer perspektivischen Seitenansicht. Hieraus wird insbesondere deutlich, dass in der Schiene 6 Aussparungen 28 zur Aufnahme der Krafteinleitungsmittel 5 und der Anfangssegmente 38 der Fingersegmente 2 eingearbeitet sind. Um die Krafteinleitungsmittel 5 und die Anfangssegmente 38 in den Aussparungen 28 zu befestigen, sind in die Schiene 6 Gewindebuchsen 33 eingearbeitet, in welche Schrauben zur Fixierung der Krafteinleitungsmittel 5 und der Anfangssegmente 38 eingeschraubt werden können. Auch sind optional Rastsitze 29 der Rastverbindung 9 zu erkennen, an welchen ein freies Ende der Palmarstütze 8 befestigbar ist. Die Palmarstütze 8 kann alternativ aber auch insgesamt einstückig mit der Schiene 6 gebildet sein. Zudem erkennt man anhand von Figur 2, dass die Schiene 6 zusätzlich einen mit der Schiene 6 einstückig gebildeten Unterarmbügel 42 aufweist zum zumindest teilweise Umgreifen des Unterarms des Patienten. Ein freies Ende des Unterarmbügels 42 wirkt vorliegend wie eine Feder oder stellt eine solche dar, welche es dem Patienten erleichtert, die Handorthese 1 bzw. die Schiene 6 an- und/oder auszuziehen. Alternativ zu dem Unterarmbügel 42 könnte bei der Schiene 6 auch ein weiterer Rastsitz 29 vorhanden sein, in den ein Befestigungsmittel - beispielsweise ein Band mit einem Rastglied oder einer Rastausnehmungeingerastet werden kann, um die Schiene 6 am Arm des Patienten zu fixieren. In der Schiene 6 verlaufen zudem die verdeckten Kanäle 30, in denen die Kopplungselemente 4, vorzugsweise in einem mehrlumigen, insbesondere doppellumigen Schlauch geführt, aufgenommen sind, die die Zug- und Druckkräfte von dem Krafteinleitungsmittel 5 auf die Fingersegmente 2 übertragen.

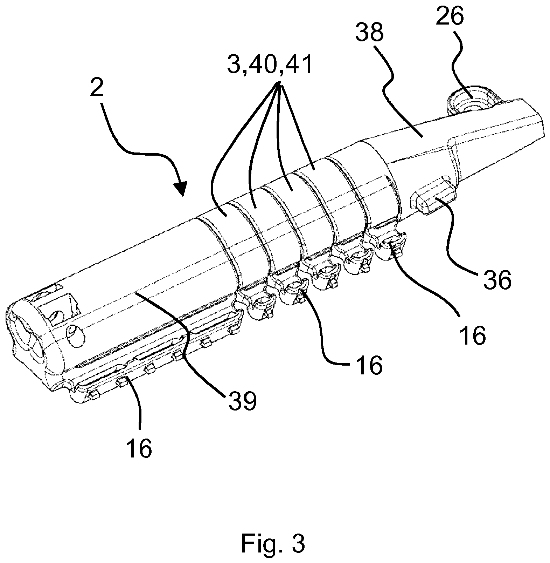

Figur 3 zeigt die perspektivische Ansicht eines der Fingersegmente 2 der Handorthese 1. Aus Figur 3 wird nochmals ersichtlich, dass das Fingersegment 2 neben den Modulsegmenten 3 auch das distale Endsegment 39 und ein proximales Anfangssegment 38 umfasst, wobei das Anfangssegment 38 an der Schiene 6 und das Endsegment 39 an dem Handschuhabschnitt 7 befestigt werden. Hierzu sind an dem Endsegment 39 Flügel 16 zum Einbetten in den Handschuhabschnitt 7 ausgebildet. Das Anfangssegment 38 weist hingegen zusätzlich neben den Flügeln 16 auch eine Schraubenaufnahme 26 auf, mit der das Fingersegment 2 mit der Schiene 6 verschraubt werden kann. Zudem können dem proximalen Anfangssegment 38 auch Einführhilfen 36 zugeordnet sein, die in korrespondierende Montageaufnahmen 37 der Schiene 9 eingeführt werden können, wodurch die Fingersegmente 2 an der Schiene 6 zusätzlich befestigt werden können (Fig. 1). Zwischen dem Anfangssegment 38 und dem Endsegment 39 sind eines oder mehrere der Modulsegmente 3 angeordnet. Die Modulsegmente 3 sind entweder als eines der Gelenksegmente 40 oder als eines der Linearsegmente 41 gebildet.

Die Gelenksegmente 40 ermöglichen eine gelenkige Verbindung, also eine um eine Schwenkachse schwenkbare Kippverbindung, die ein Abwinkeln ermöglicht, während die Linearsegmente 41 eine translatorische, aber unverschwenkbare Verstellung der Segmente 38, 39, 40, 41 relativ zueinander ermöglichen. Um die Länge der Fingersegmente 2 an die Länge der Finger des Patienten anpassen zu können, und um die gewünschte Bewegbarkeit der Finger herzustellen, ist folglich eine Variation der Anzahl der Gelenksegmente 40 und der Anzahl der Linearsegmente 41 möglich und erforderlich.

Die verschiedenen Segmente 38, 39, 40, 41 sind miteinander, d.h. mit dem jeweils benachbarten Segment 38, 39, 40, 41 über eine Bajonettverbindung 10 miteinander koppelbar oder gekoppelt. Die Funktionsweise der jeweiligen Bajonettverbindung 10 wird dabei folgend anhand der Figuren 4 bis 7 erläutert.

Figur 4a bis 4d zeigen mehrere Ansichten des Anfangssegments 38. Wie weiter oben bereits erläutert wurde, weist das Anfangssegment 38 seitlich die Einführhilfe 36 zur Befestigung an der Schiene 6 und jeweils seitlich zwei Flügel 16 zum Einbetten des Anfangssegments 38 in das Silikon des Handschuhabschnitts 7 auf. Die Flügel 16 aller Segmente 38, 39, 40, 41 weisen jeweils mindestens eine Öffnung 17 auf und sind zur Bildung eines Widerhakens 18 hakenförmig gebildet, wodurch die Segmente 38, 39, 40, 41 besser in dem aus Silikon gebildeten Handschuhabschnitt 7 verankern. Um überschüssiges Silikonmaterial beim Einbetten der Segmente 38, 39, 40, 41 in den Handschuhabschnitt 7 abstreifen und entfernen zu können, sind zudem Abstreifhilfen 34 vorgesehen. Beim Einbetten kann somit überschüssiges Silikonmaterial einfach - beispielsweise mittels eines auf den Abstreifhilfen 34 aufgelegten Spatels - abgestreift werden.

Anhand der schienenzugewandten Ansicht des Anfangssegments 38 in Fig. 4c erkennt man zudem, dass das Anfangssegment 38 eine Durchführung 15 für die Kopplungselemente 4 aufweist. In der Durchführung 15 ist vorzugsweise ein mehrlumiger, insbesondere doppellumiger Schlauch angeordnet oder hindurchgeführt, in dem jeweils ein als Draht - vorzugsweise ein Nitinoldraht - gebildetes Kopplungselement 4 aufgenommen ist. Fig. 4d zeigt die schienenabgewandte Ansicht des Anfangssegments 38, welches mit einem der weiteren Modulsegmente 3 - entweder einem Gelenksegment 40 oder einem Linearsegment 41 - mittels der Bajonettverbindung 10 koppelbar ist. Hierzu weist das Anfangssegment 38 an einer der Schiene 6 abgewandten Stirnseite 35 vorliegend mindestens einen Bajonetthaken 14, vorzugsweise aber vier Bajonetthaken 14 auf, die in mindestens eine, vorliegend in vier Bajonettkulissen 14 eines benachbarten Modulsegments 3 eingreifen können.

Figuren 5a bis 5d zeigen mehrere Ansichten des Gelenkssegments 40. Das Gelenksegment 40 weist analog zum Anfangssegment 38 ebenfalls seitlich jeweils einen Flügel 16 auf. Figuren 5b bis 5d verdeutlichen zudem, dass an einer der Stirnseiten eine zur Anzahl der Bajonetthaken 14 korrespondierende Anzahl an Bajonettkulissen 12 ausgebildet sind. Die Bajonetthaken 14 dienen dem Eingriff in die Bajonettkulissen 12 eines benachbarten Segments 38, 39, 40, 41.

An derjenigen Stirnseite, an der die Bajonetthaken 14 ausgebildet sind, sind eine an die Anzahl der Bajonetthaken 14 angepasste Anzahl an Vorsprüngen 21 vorhanden, die von den Bajonettkulissen 12 eines benachbarten Segments 38,39,40 im montierten Zustand hintergriffen werden.

Während an derjenigen Stirnseite, an der die Bajonettkulissen 12 ausgebildet sind, insgesamt vier Aufnahmen 20 vorhanden sind, die die Vorsprünge 21 des benachbarten Segments 39,40,41 im montierten Zustand ebenfalls hintergreifen.

Beim Gelenksegment 40 sind die neben den Bajonettkulissen 12 vorhandenen Vorsprünge 21, zumindest aber die beiden unteren Vorsprünge 21a, mit einer bezüglich der Längsachse des Gelenksegments 40 geneigt verlaufenden Vorsprungsfläche 45 gebildet. Die Vorsprungsflächen 45 der Vorsprünge 21 laufen dabei von außen nach innen aufeinander zu. Diese geneigten Vorsprungsflächen 45 ermöglichen die Kippbewegung.

Dabei entspricht die Tiefe, mithin also die entlang der Längsachse axiale Erstreckung der Vorsprünge 21 (insbesondere der beiden unteren Vorsprünge 21a), gleich oder annähernd gleich der Tiefe, mithin der entlang der Längsachse axialen Erstreckung der Aufnahmen 20a (insbesondere der beiden unteren Aufnahme 20a). Analog dazu entspricht die Tiefe der (unteren) Bajonetthaken 14, 14a annähernd der Tiefe der (unteren) Bajonettkulissen 12, 12a.

Beim Verbinden eines Gelenksegments 40 mit einem benachbarten Segment 38, 39, 40, 41 werden die Bajonetthaken 14 des benachbarten Segments 38, 39, 40, 41 durch eine Einführöffnung 22 in die Bajonettkulisse 12 des Gelenksegments 40 eingeführt und gegeneinander verdreht. Durch die abgewinkelte Ausbildung der unteren Vorsprünge 21a des Gelenksegments 40 und dadurch, dass die unteren Bajonetthaken 14a sowie die unteren Vorsprünge 21a kaum Spiel in der unteren Aufnahme 20a und in der unteren Bajonettkulisse 12a aufweisen, wird eine translatorische Bewegung unterbunden, wobei aufgrund der geneigt verlaufenden Vorsprungsflächen 45 der Gelenksegmente 40 eine Kippbewegung möglich bleibt.

Bezugnehmend zu Figuren 6a bis 6d ist folgendes festzustellen: Beim Linearsegment 41 sind die neben den Bajonettkulissen 12 vorhandenen Vorsprünge 21, zumindest aber die beiden unteren Vorsprünge 21a, mit einer bezüglich der Längsachse des Linearsegments 41 parallel oder koaxial verlaufenden Vorsprungsfläche 45 gebildet. Die Vorsprungsflächen 45 der Vorsprünge 21 laufen dabei alle koaxial zu einander, so dass es dem Linearsegment 41 - anders als dem Gelenksegment 40 - an einer geneigt verlaufenden Vorsprungsfläche 45 fehlt. Diese Vorsprungsflächen 45 ermöglichen die Translation.

Die (unteren) Aufnahmen 20, 20a bei dem Linearsegment 42 weisen eine größere Tiefe auf, als die Tiefe der (unteren) Vorsprünge 21, 21a. Greifen die Bajonetthaken 14 des Linearsegments 41 in die Bajonettkulissen 12 des benachbarten Segments 38, 39, 40, 41 ein, so haben der (untere) Bajonetthaken 14, 14a und der (obere) Bajonetthaken 14, 14b annähernd genauso viel Spiel. Dies ermöglicht eine translatorische Verstellung. In anderen Worten ist die Tiefe der (unteren) Aufnahmen 20, 20a der Linearsegmente 41 und die der (oberen) Aufnahmen 20, 20b annähernd gleich. Zudem ist die Tiefe der Vorsprünge 21 der Segmente 38, 39, 40, 41 kleiner als die Tiefe der Aufnahmen 20 der Linearsegmente 41. Demgegenüber sind die Tiefe der unteren Aufnahmen 20, 20a des Gelenksegments 40 kleiner als die oberen Aufnahmen 20, 20b des Gelenksegments 40 und die Tiefe der unteren Aufnahme 20a entsprechen annähernd der Tiefe der unteren Vorsprünge 21a.

Figuren 7a bis 7d zeigen mehrere Darstellungen des Endsegments 39. Im Unterschied zu den übrigen Segmenten sind am Endsegment 39 seitlich jeweils eine Mehrzahl von Flügeln 16, vorliegend jeweils fünf Flügel 16 ausgebildet. Insbesondere der Fig. 7c ist zu entnehmen, dass am Endsegment 39 eine Bajonettkulisse 12 ausgebildet ist. Zudem wird anhand von Fig. 7b deutlich, dass die Kopplungselemente 4 zur Kopplung mit den Krafteinleitungsmitteln 5 mittels einer Schraubverbindung 32 gesichert werden können. Bei allen Segmenten 38, 39, 40, 41 ist die Durchführung 15 gegenüber der Bajonettverbindung 10, insbesondere gegenüber den Bajonetthaken 14 nach oben versetzt angeordnet, um eine bessere Kraftübertragung zu gewährleisten. Das Endsegment 39 weist zudem eine mit einem nicht näher dargestellten Cover verschließbare Wartungsöffnung 43 auf, um die Wartung der Kopplungselemente 4 zu vereinfachen. Darüber hinaus sind im Endsegment 38 Hohlräume 44 ausgebildet zur Aufnahme von Silikon, um das Einbetten des Endsegments 39 in den Handschuhabschnitt 7 zu verbessern.

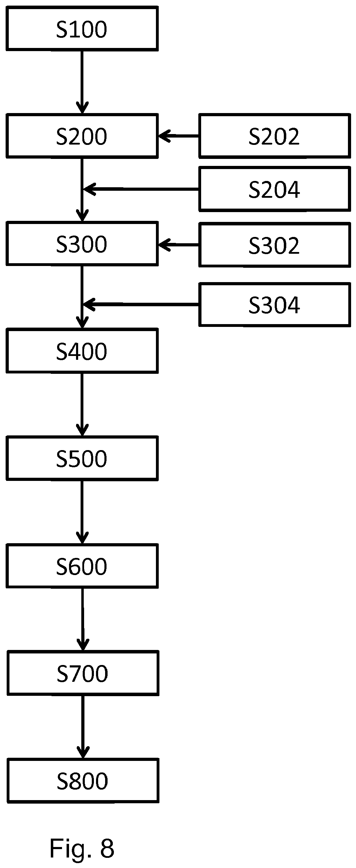

In Figur 8 wird im Folgenden das Verfahren zur Herstellung einer Handorthese 1 näher dargestellt. Zunächst wird ein Abdruck mindestens eines Fingers einer Patientenhand, vorzugsweise jedoch aller Finger oder der gesamten Patientenhand, sowie eines Unterarms erstellt (Schritt: S100). Bevorzugt wird dazu ein Gipsabdruck erstellt, wobei der Gipsabdruck vorliegend mindestens zweiteilig durch eine Oberschale und eine Unterschale gebildet ist. Er kann also mehrteilig vorliegen. Der Abdruck der Fingers und/oder der Patientenhand kann getrennt vom Abdruck des Unterarms erstellt werden. Es ist jedoch auch möglich, einen monolithischen Abdruck von Patientenhand und Unterarm zu erstellen. Der Abdruck wird dabei vorzugsweise von der Patientenhand in einer gebeugten Stellung erstellt. Das Handgelenk weist dabei vorzugsweise eine Extension von 10 Grad bis 30 Grad auf, die MCP-Gelenke sind gestreckt, die PIP- und DIP-Gelenke sind um ungefähr 20 Grad bis 50 Grad gebeugt, wobei sich der Daumen in einer Oppositionsstellung oder in einer Lateralstellung befindet.

Die Finger sind gespreizt, d.h. zwischen dem Zeige-, dem Mittel-, und dem Ringfinger ist jeweils ein Spalt oder Abstand von mindestens 5 Millimeter (mm) auf Höhe des PIP. Der Ringfinger und der kleine Finger können dabei näher als 5 mm beieinanderliegen. Um diese gebeugte Stellung der Patientenhand komfortabler und sicherer halten zu können, ist es daher vorgesehen, dass die gebeugte Stellung der Patientenhand mittels einer Positioniervorrichtung gehalten wird. Die Positioniervorrichtung kann als ein Klotz, ein Keil oder mehrere miteinander kombinierbare Keile oder Klötze gebildet sein. In einer besonders einfachen Ausführungsform wird die Hand um oder auf einen Ball gelegt.

Anhand des Abdrucks, der in Form eines Negativs vorliegt, wird im vorliegenden Falle zunächst eine Nachbildung des mindestens einen Fingers, vorzugsweise der Patientenhand erstellt, womit ein physisches Modells für die weiteren Verfahrensschritte vorliegt. Im Anschluss wird der Fingerabschnitt, oder auch der Handschuhabschnitt 7 anhand der Nachbildung des mindestens einen Fingers erstellt (S200), indem mehrere Lagen von Silikon auf den die Nachbildung aufgetragen werden (S202). Dabei wird Silikon mit unterschiedlichen Härtegraden zum Erstellen des Fingerabschnitts oder des Handschuhabschnitts 7 verwendet. Insbesondere im Bereich der Einbettung des mindestens einen Fingersegments 2 wird ein erstes Silikon verwendet und im Bereich eines PIP-Gelenks und/oder eines DIP-Gelenks des Fingersegments 2 wird ein zweites Silikon verwendet, wobei der erste Silikon einen weicheren Härtegrad aufweist als der zweite Silikon. Das erste Silikon weist beispielsweise eine Härte zwischen 25 und 45 Shore A, vorzugsweise 35 Shore A auf. Das zweite Silikon weist beispielsweise eine Härte von zwischen 55 und 75 Shore A, vorzugsweise 65 Shore A auf. Dies ermöglicht es, den Fingerabschnitt oder den Handschuhabschnitt 7 in Bereichen höherer Belastung steifer und in Bereichen, in denen eine Beugung oder Streckung erfolgt, weicher zu gestalten. Um eine individuell an den Patienten angepasste Handorthese 1 zu erstellen, wird die Länge der Fingersegmente 2 an die Länge der Finger des Patienten angepasst (S204). Dies erfolgt durch das Anordnen und Koppeln von einem oder von mehreren Modulsegmenten 3 zwischen dem mit der Schiene 6 gekoppelten Anfangssegment 38 und dem mit dem Krafteinleitungsmittel 5 gekoppelten Endsegment 39. Die einzelnen Segmente 38, 39, 40, 41 werden dabei über die oben genannten Bajonettverbindungen 10 miteinander gekoppelt. Die Länge und die Bewegbarkeit wird insbesondere auch dadurch angepasst, dass das Modulsegment 3 als ein Linearsegment 41 oder als ein Gelenkssegment 40 gebildet ist, und dass die Länge des mindestens einen Fingersegments 2 durch eine Kombination von einem oder von mehreren Gelenkssegmenten 40 mit einem oder mit mehreren Linearsegmenten 41 angepasst wird. Die Anzahl der Linearsegmente 41 und der Gelenksegmente 40 bestimmt somit die Länge und die Bewegbarkeit der einzelnen Fingerglieder des Fingersegments 2.

Anschließend wird das mindestens eine Fingersegment 2 in die der Handoberfläche entsprechende Seite des Fingerabschnitts oder des Handschuhabschnitts 7 eingebettet (S300). Dies erfolgt vorzugsweise dadurch, dass die Fingersegmente 2 mit den Flügeln 16 im Silikonmaterial verankert werden, diese also vom Silikon umgeben sind, wobei dabei das Silikon die Öffnungen 17 durchdringt. Die Widerhaken 18 bieten dabei zusätzliche Stabilität. Hierzu wird auf die Unterseite der Flügel 16 bevorzugt mittels einer Silikonstanze Silikon aufgetragen und in die Hohlräume 44 und Öffnungen 17 gedrückt. Die Fingersegmente 2 werden dann auf den Fingerabschnitt oder den Handschuhabschnitt 7 aufgedrückt und zusätzlich nochmals von der Oberseite mit Silikon gefüllt. Überschüssiges Silikon, das über die Abstreifhilfen 34 übersteht, kann mittels eines Spatels entfernt werden. Auch hier können Silikone mit unterschiedlichen Härtegraden verwendet werden.

Gleichzeitig, zeitlich vorher oder auch danach wird das PIP-Gelenk und/oder eines der DIP-Gelenke durch zusätzliches Einbetten von Material in das Silikon stabilisiert und/oder versteift, wobei das Material einen höheren Härtegrad als das verwendete Silikon aufweist (S302).

Das Silikon des Fingerabschnitts oder des Handschuhabschnitts 7 wird daraufhin mittels Hitzeeinwirkung, bevorzugt in einem Ofen, vernetzt.

Auf die Nachbildung und auf den Fingerabschnitt oder den Handschuhabschnitt 7 kann nun optional eine Polsterung aufgebracht werden (S304). In einem weiteren Schritt wird nun die Physiologie des Unterarms des Patienten mittels eines 3D-Scans der auf dem Abdruck basierenden Nachbildung des Unterarms zusammen mit dem Fingerabschnitt oder dem Handschuhabschnitt 7 und der eventuell aufgebrachten Polsterung erfasst und dadurch ein digitales 3D-Modell erstellt (S400). Alternativ, wenn auf dem Fingerabschnitt oder dem Handschuhabschnitt 7 noch keine Polsterung aufgebracht ist, kann die Physiologie des Unterarms auch anhand der auf dem Abdruck basierenden Nachbildung des Unterarms zusammen mit dem Fingerabschnitt oder dem Handschuhabschnitt 7 allein, mithin polsterungsfrei erfasst werden und ein entsprechendes digitales 3D-Modell erstellt werden. Dem so erstellten digitalen 3D Modell kann dann ein Offset hinzugefügt werden, der für das spätere Aufbringen einer Polsterung bestimmt ist. Der Offset kann dabei der Dicke der geplanten Polsterung entsprechen oder kleiner als die geplante Polsterung sein, um die Handorthese 1 später enganliegender an die Patientenhand anzupassen.

Wurde die Polsterung noch nicht auf den Fingerabschnitt oder den Handschuhabschnitt 7 aufgebracht, so kann dies auch nach dem Erstellen des 3D-Modells und vor oder nach dem Erzeugen einer Schiene 6 auf Grundlage des erstellten digitalen 3D-Modells erfolgen. Dabei wird bevorzugt die gesamte Fläche des Fingerabschnitts oder des Handschuhabschnitts 7 und der Schiene 6, die mit der Hand bzw. dem Unterarm des Patienten in Berührung kommen, mit einer Polsterung versehen. Die Schiene 6 kann dabei auf Grundlage des 3D-Modells bevorzugt mittels eines generativen Fertigungsverfahrens erzeugt werden (S500), insbesondere mittels eines 3D-Druckverfahrens. Bei dieser Herstellung werden bereits die für Befestigung der Fingersegmente 2 sowie der Krafteinleitungsmittel 5 benötigten Aussparungen 28 und die Kanäle 30 eingearbeitet. Die Gewindebuchsen 33 werden dann nachträglich in die Schiene 6 eingearbeitet.

Dem digitalen 3D-Modell kann optional auch mindestens eine Palmarstütze 8 und/oder mindestens ein Unterarmbügel 42 hinzugefügt werden, so dass die mittels eines generativen Fertigungsverfahrens erzeugte Schiene 6 auch eine Palmarstütze 8 und/oder einen Unterarmbügel 42 umfasst.

In einem weiteren Schritt wird das Krafteinleitungsmittel 5 an der Schiene 6 in einer der Aussparungen 28 befestigt (S600). Gleichzeitig, zuvor oder nachfolgend wird das Anfangssegment 38 des mindestens einen Fingersegments 2 an einem distalen Ende in den dafür vorgesehenen Aussparungen 28 der Schiene 6 befestigt (S700). Hierzu werden die an den Anfangssegmenten 38 ausgebildeten Einführhilfen 36 in die Montageaufnahmen 37 der Schiene 6 eigeführt und mit der Schraubenaufnahme 26 an der Schiene 6 festgeschraubt. Dies erfolgt für alle Fingersegmente 2. Schließlich werden die Krafteinleitungsmittel 5 mit den Fingersegmenten 2 vorzugsweise im Bereich des distalen Endes, also an den Endsegmenten 39 gekoppelt (S800). Es sei an dieser Stelle ergänzend angemerkt, dass die Schritte S600, S700 und S800 erfindungsgemäß in beliebiger Reihenfolge oder auch gleichzeitig durchgeführt werden können.

Um eine glatte Oberfläche der Handorthese 1 bereitzustellen, wird in einem abschließenden Verfahrensschritt eine nicht näher dargestellte Abdeckung in Form einer Abdeckhaube oder in Form eines Cover auf die dem Unterarm abgewandte Oberfläche der Schiene 6 aufgebracht. Die Abdeckung deckt dabei insbesondere den Bereich der Aussparungen 28 ab. Die Abdeckung wird mit der Schiene 6 verschraubt und verdeckt und schützt dann die zumeist empfindlichen Krafteinleitungsmittel 5 bzw. Servomotoren.

### BEZUGSZEICHENLISTE

- 1: Handorthese
- 2: Fingersegment
- 3: Modulsegment
- 4: Kopplungselement
- 5: Krafteinleitungsmittel
- 6: Schiene
- 7: Handschuhabschnitt
- 8: Palmarstütze
- 9: Rastverbindung
- 10: Bajonettverbindung
- 12: Bajonettkulisse
- 12a: untere Bajonettkulisse
- 12b: obere Bajonettkulisse
- 14: Bajonetthaken
- 14a: untere Bajonetthaken
- 14b: oberer Bajonetthaken
- 15: Durchführung
- 16: Flügel
- 17: Öffnung
- 18: Widerhaken
- 20: Aufnahme
- 20a: untere Aufnahme
- 20b: obere Aufnahme
- 21: Vorsprung
- 21a: unterer Vorsprung
- 21b: oberer Vorsprung
- 22: Einführöffnung
- 26: Schraubenaufnahme
- 28: Aussparung
- 29: Rastsitz
- 30: Kanal
- 32: Schraubverbindung
- 33: Gewindebuchse
- 34: Abstreifhilfe
- 36: Einführhilfe
- 37: Montageaufnahme
- 38: Anfangssegment
- 39: Endsegment
- 40: Gelenksegment
- 41: Linearsegment
- 42: Unterarmbügel
- 43: Wartungsöffnung
- 44: Hohlraum
- 45: Vorsprungsfläche

## Patentansprüche

1. Verfahren zur Herstellung einer Handorthese (1), die einen Fingerabschnitt zur Aufnahme einer Patientenhand, eine Schiene (6) zum zumindest teilweise umgreifen eines Unterarms eines Patienten und mindestens ein mit einem Krafteinleitungsmittel (5) gekoppeltes Fingersegment (2) aufweist zum Beugen und/oder Strecken mindestens eines Fingers einer Patientenhand, umfassend die Schritte:
- Erstellen eines Abdrucks mindestens eines Fingers einer Patientenhand und zumindest eines Teils eines Unterarms des Patienten (S100),
- Erstellen mindestens eines Fingerabschnitts anhand des Abdrucks oder anhand einer auf dem Abdruck basierenden Nachbildung des mindestens eines Fingers der Patientenhand (S200),
- Einbetten von mindestens einem Fingersegment (2) in die der Handoberfläche entsprechende Seite des Fingerabschnitts (S300),
- Erfassen der Physiologie des Unterarms des Patienten mittels mindestens einer Aufnahme von dem Abdruck oder von einer auf dem Abdruck basierenden Nachbildung des Unterarms zusammen mit dem Fingerabschnitt und Erstellen eines digitalen 3D-Modells anhand der mindestens einen Aufnahme (S400),
- Erzeugen einer Schiene (6) auf Grundlage des erstellten digitalen 3D-Modells (S500),
- Befestigen von mindestens einem Krafteinleitungsmittel (5) auf oder in die Schiene (6) (S600),
- Befestigen eines proximalen Endes des mindestens einen Fingersegments (2) an einem distalen Ende der Schiene (6) (S700) und
- Koppeln des mindestens einen Krafteinleitungsmittels (5) mit dem mindestens einen Fingersegment (2) (S800).

2. Verfahren nach Anspruch 1, **dadurch gekennzeichnet, dass** der Abdruck unter einer gebeugten Stellung der Patientenhand erstellt wird.

3. Verfahren nach Anspruch 2, **dadurch gekennzeichnet, dass** die gebeugte Stellung mittels einer Positioniervorrichtung gehalten wird.

4. Verfahren nach einem der Ansprüche 1 bis 3, **dadurch gekennzeichnet, dass** der Fingerabschnitt mittels eines Auftragens von Silikon auf den Abdruck oder die auf dem Abdruck basierende Nachbildung erstellt wird (S202).

5. Verfahren nach Anspruch 4, **dadurch gekennzeichnet, dass** verschiedene Silikone mit unterschiedlichen Härtegraden zum Erstellen des Fingerabschnitts verwendet werden und dass insbesondere im Bereich der Einbettung des mindestens einen Fingersegments (2) Silikon mit einem weicheren Härtegrad und im Bereich eines PIP-Gelenks und/oder eines DIP-Gelenks des Fingersegments (2) mit einem härteren Härtegrad verwendet wird.

6. Verfahren nach Anspruch 4 oder 5, umfassend die Schritte
- Stabilisieren und/oder Versteifen eines PIP-Gelenks und/oder eines DIP-Gelenks durch zusätzliches Einbetten von einem Material in das Silikon, welches gegenüber dem Silikon einen höheren Härtegrad aufweist (S302).

7. Verfahren nach einem der Ansprüche 4 bis 6, **dadurch gekennzeichnet, dass** das mindestens eine Fingersegment (2) in das Silikon mittels einer Silikonstanze eingebettet wird.

8. Verfahren nach einem der Ansprüche 1 bis 7, umfassend den folgenden Schritt:
- Anpassen der Länge des mindestens einen Fingersegments (2) an die Länge eines Fingers des Patienten durch Anordnen und Koppeln von einem oder von mehreren Modulsegmenten (3) zwischen einem mit der Schiene (6) gekoppelten Anfangssegment (38) und einem mit dem Krafteinleitungsmittel (5) gekoppelten Endsegment (39) (S204)

9. Verfahren nach Anspruch 8, **dadurch gekennzeichnet, dass** das Modulsegment (3) als ein Linearsegment (41) oder als ein Gelenksegment (40) gebildet ist, und dass die Länge des mindestens einen Fingersegments (2) durch eine Kombination von mehreren Modulsegmenten (3), insbesondere durch eine Kombination von einem oder von mehreren Gelenksegmenten (40) mit einem oder mit mehreren Linearsegmenten (41), angepasst wird.

10. Verfahren nach einem der Ansprüche 1 bis 9, **dadurch gekennzeichnet, dass** die Physiologie des Unterarms und dadurch das digitale 3D-Modell mittels der mindestens einen Aufnahme des Abdrucks oder der auf dem Abdruck basierenden Nachbildung des Unterarms zusammen mit dem Fingerabschnitt und mit einer auf zumindest Teilen des Fingerabschnitts und/oder zumindest Teilen des Abdrucks oder der mindestens einen auf dem mindestens einen Abdruck basierenden Nachbildung aufgebrachten Polsterung erfasst wird.

11. Verfahren nach einem der Ansprüche 1 bis 9, **dadurch gekennzeichnet, dass** dem digitalen 3D-Modell ein Offset für das Aufbringen einer Polsterung hinzugefügt wird.

12. Verfahren nach einem der Ansprüche 1 bis 11, umfassend den Schritt:
- Aufbringen einer Polsterung auf zumindest einen Teil eines Handrückens des Fingerabschnitts und/oder auf zumindest einen Teil der Schiene (6) (S304).

13. Verfahren nach einem der Ansprüche 1 bis 12, **dadurch gekennzeichnet, dass** dem digitalen 3D-Modell eine Palmarstütze (8) und/oder ein Unterarmbügel (42) hinzugefügt wird.

14. Verfahren nach einem der Ansprüche 1 bis 13, **dadurch gekennzeichnet, dass** vor dem Erfassen der Physiologie des Unterarms mittels der mindestens einen Aufnahme mindestens eine Markierungslinie oder eine Mehrzahl von Markierungspunkten zur Festlegung einer späteren Kontur der Schiene (6) auf den Abdruck oder auf die auf dem Abdruck basierende Nachbildung aufgebracht wird.

## Claims

1. A method of producing a hand orthosis (1) having a finger segment for receiving a patient's hand, a splint (6) for at least partially embracing a patient's forearm, and at least one finger segment (2) coupled to a force-introducing device (5) for bending and/or stretching at least one finger of a patient's hand, comprising the steps of:
- Making an impression of at least one finger of a patient's hand and at least a part of a forearm of the patient (S100),
- creating at least one finger segment from the impression or from a replica of the at least one finger of the patient hand based on the impression (S200),
- embedding at least one finger segment (2) in the side of the finger segment (S300) corresponding to the surface of the hand,
- detecting the physiology of the patient's forearm by means of at least one image from the impression or from a replica of the forearm based on the impression together with the finger segment, and creating a digital 3D model based on the at least one image (S400),
- creating a splint (6) based on the created 3D digital model (S500),
- attaching at least one force-introducing device (5) onto or into the splint (6) (S600),
- attaching a proximal end of the at least one finger segment (2) to a distal end of the splint (6) (S700), and
- coupling the at least one force-introducing device (5) to the at least one finger segment (2) (S800).

2. The method according to claim 1, wherein the impression is made under a bent position of the patient's hand.

3. The method according to claim 2, wherein the bent position is held by means of a positioning device.

4. The method according to any one of claims 1 to 3, wherein the finger segment is created by applying silicone to the impression or the replica based on the impression (S202).

5. The method according to claim 4, wherein different silicones with different degrees of hardness are used to create the finger segment and in particular silicone with a softer degree of hardness is used in the region of the embedding of the at least one finger segment (2) and silicone with a harder degree of hardness is used in the region of a PIP-hinge and/or a DIP-hinge of the finger segment (2).

6. The method according to claim 4 or 5, comprising the steps of
- Stabilizing and/or reinforcing a PIP-hinge and/or a DIP-hinge by additionally embedding in the silicone a material which has a higher degree of hardness than the silicone (S302).

7. The method according to one of claims 4 to 6, wherein at least one finger segment (2) is embedded in the silicone by means of a silicone punch.

8. The method according to any one of claims 1 to 7, comprising the following step:
- Adapting the length of the at least one finger segment (2) to the length of a finger of the patient by arranging and coupling one or more module members (3) between an initial segment (38) coupled to the splint (6) and an end segment (39) (S204) coupled to the force-introducing device (5).

9. The method according to claim 8, wherein the module member (3) is formed as a linear segment (41) or as an articulated segment (40), and that the length of the at least one finger segment (2) is adapted by a combination of several module members (3), in particular by a combination of one or more articulated segments (40) with one or more linear segments (41).

10. The method according to any one of claims 1 to 9, wherein the physiology of the forearm and thereby the 3D digital model is acquired by means of the at least one image of the impression or the replica of the forearm based on the impression together with the finger segment and with padding applied to at least parts of the finger segment and/or at least parts of the impression or the at least one replica based on the at least one impression.

11. The method according to any one of claims 1 to 9, wherein an offset for the application of padding is added to the digital 3D model.

12. The method according to any one of claims 1 to 11, comprising the step:
- Applying padding to at least a portion of a back of the hand of the finger segment and/or to at least a part of the splint (6) (S304).

13. The method according to any one of claims 1 to 12, wherein a palmar support (8) and/or a forearm support (42) is added to the digital 3D model.

14. The method according to any one of claims 1 to 13, wherein, before the physiology of the forearm is recorded by means of the at least one recording, at least one marking line or a plurality of marking points is applied to the impression or to the replica based on the impression for defining a future contour of the splint (6).

## Revendications

1. Procédé de fabrication d'une orthèse de la main (1) qui présente une section pour doigts permettant d'accueillir une main de patient, une gouttière (6) permettant d'entourer au moins partiellement un avant-bras d'un patient et au moins un segment pour doigt (2) couplé à un moyen d'introduction de force (5) afin de plier et/ou étirer au moins un doigt d'une main de patient, comprenant les étapes consistant à :
- réaliser (S100) une empreinte d'au moins un doigt d'une main de patient et d'au moins une partie d'un avant-bras du patient,
- réaliser (S200) au moins une section pour doigts en se basant sur l'empreinte ou en se basant sur une réplique du au moins un doigt de la main de patient basée sur l'empreinte,
- incruster (S300) au moins un segment pour doigt (2) dans le côté de la section pour doigts qui correspond à la surface de la main,
- acquérir (S400) la physiologie de l'avant-bras du patient au moyen d'au moins un enregistrement de l'empreinte ou d'une réplique de l'avant-bras basée sur l'empreinte conjointement avec la section pour doigts et créer un modèle numérique 3D en se basant sur le au moins un enregistrement,
- fabriquer (S500) une gouttière (6) en se basant sur le modèle numérique 3D créé,
- fixer (S600) au moins un moyen d'introduction de force (5) sur ou dans la gouttière (6),
- fixer (S700) une extrémité proximale du au moins un segment pour doigt (2) à une extrémité distale de la gouttière (6), et
- coupler (S800) le au moins un moyen d'introduction de force (5) à l'au moins un segment pour doigt (2).

2. Procédé selon la revendication 1, **caractérisé en ce que** l'empreinte est réalisée avec la main de patient en position fléchie.

3. Procédé selon la revendication 2, **caractérisé en ce que** la position fléchie est maintenue au moyen d'un dispositif de positionnement.

4. Procédé selon l'une quelconque des revendications 1 à 3, **caractérisé en ce que** la section pour doigts est réalisée (S202) au moyen d'une application de silicone sur l'empreinte ou sur la réplique basée sur l'empreinte.

5. Procédé selon la revendication 4, **caractérisé en ce que** différents silicones présentant différents degrés de dureté sont utilisés pour la réalisation de la section pour doigts, et en particulier **en ce que** du silicone présentant un degré de dureté inférieur est utilisé dans la région de l'incrustation du au moins un segment pour doigt (2) et du silicone présentant un degré de dureté supérieur est utilisé dans la région d'une articulation IPP et/ou d'une articulation IPD du segment pour doigt (2).

6. Procédé selon la revendication 4 ou 5, comprenant l'étape consistant à
- stabiliser et/ou raidir (S302) une articulation IPP et/ou une articulation IPD grâce à une incrustation supplémentaire dans le silicone d'un matériau, lequel présente un degré de dureté supérieur à celui du silicone.

7. Procédé selon l'une quelconque des revendications 4 à 6, **caractérisé en ce que** le au moins un segment pour doigt (2) est incrusté dans le silicone au moyen d'une estampeuse à silicone.

8. Procédé selon l'une quelconque des revendications 1 à 7, comprenant l'étape suivante :
- adapter (S204) la longueur du au moins un segment pour doigt (2) à la longueur d'un doigt du patient grâce à l'agencement et au couplage d'un ou de plusieurs segment(s) de module (3) entre un segment initial (38) couplé à la gouttière (6) et un segment final (39) couplé au moyen d'introduction de force (5).

9. Procédé selon la revendication 8, **caractérisé en ce que** le segment de module (3) est réalisé sous la forme d'un segment linéaire (41) ou d'un segment d'articulation (40), et **en ce que** la longueur du au moins un segment pour doigt (2) est adaptée grâce à une combinaison de plusieurs segments de module (3), en particulier grâce à une combinaison d'un ou de plusieurs segment(s) d'articulation (40) avec un ou plusieurs segment(s) linéaire(s) (41).

10. Procédé selon l'une quelconque des revendications 1 à 9, **caractérisé en ce que** la physiologie de l'avant-bras, et par conséquent le modèle numérique 3D, est acquise au moyen du au moins un enregistrement de l'empreinte ou de la réplique de l'avant-bras basée sur l'empreinte conjointement avec la section pour doigts et avec un rembourrage appliqué sur au moins des parties de la section pour doigts et/ou au moins des parties de l'empreinte ou de la au moins une réplique basée sur la au moins une empreinte.

11. Procédé selon l'une quelconque des revendications 1 à 9, **caractérisé en ce qu'**un décalage pour l'application d'un rembourrage est ajouté au modèle numérique 3D.

12. Procédé selon l'une quelconque des revendications 1 à 11, comprenant l'étape consistant à :
- appliquer (S304) un rembourrage sur au moins une partie d'un dos de main de la section pour doigts et/ou sur au moins une partie de la gouttière (6).

13. Procédé selon l'une quelconque des revendications 1 à 12, **caractérisé en ce qu'**un support palmaire (8) et/ou un support d'avant-bras (42) est/sont ajouté(s) au modèle numérique 3D.

14. Procédé selon l'une quelconque des revendications 1 à 13, **caractérisé en ce qu'**au moins un trait de marquage ou une pluralité de points de marquage est/sont appliqué(s) sur l'empreinte ou sur la réplique basée sur l'empreinte avant l'acquisition de la physiologie de l'avant-bras au moyen du au moins un enregistrement afin de déterminer un contour ultérieur de la gouttière (6).
